# EUROPEAN PATENT SPECIFICATION

(11) **EP 1 905 443 B1**
(45) Date of publication and mention of the grant of the patent: **15.12.2010**
(21) Application number: 06766477.1
(22) Date of filing: 08.06.2006
(51) Int. Cl.: A61K 31/7016, A61P 41/00, C07H 3/04

(54) **LIQUID COMPRISING TREHALOSE FOR USE IN PREVENTING TISSUE ADHESION**
FLÜSSIGKEIT ENTHALTEND TREHALOSE ZUR VERWENDUNG BEI DER PRÄVENTION VON GEWEBEADHÄSION
LIQUIDE COMPRENANT DU TREHALOSE POUR LA PRÉVENTION DE L'ADHÉSION TISSULAIRE

(30) Priority: 08.06.2005 JP 2005168744
(43) Date of publication of application: 02.04.2008
(73) Proprietor: OTSUKA PHARMACEUTICAL FACTORY, INC., Naruto-shi, Tokushima 772-8601 (JP); KABUSHIKI KAISHA HAYASHIBARA SEIBUTSU KAGAKU KENKYUJO, Okayama-shi, Okayama 700-0907 (JP); The University of Tokyo, Bunkyo-ku, Tokyo 113-8654 (JP)
(72) Inventor: SUZUKI, Shigeki, 1350061 (JP); MIWA, Yoshikatsu K.K.K. Seibutsu Kagaku Kenkyujo, Okayama-shi, Okayama 7000907 (JP); SASAKI, Nobuo c/o THE UNIVERSITY OF TOKYO, Tokyo 1138654 (JP); TEI, Yuichi c/o THE UNIVERSITY OF TOKYO, Tokyo 1138654 (JP)
(74) Representative: Grünecker, Kinkeldey, Stockmair & Schwanhäusser Anwaltssozietät
(86) International application number: PCT/JP2006/311501
(87) International publication number: WO 2006/132310

(56) References cited:
- EP-A- 1 512 404
- WO-A-00/07634
- WO-A-00/64254
- WO-A-00/72872
- WO-A-93/15234
- WO-A-2004/098285
- WO-A-2005/030248
- WO-A-2006/026170
- WO-A1-2004/071472
- JP-A- 2000 512 625
- JP-A- 2006 188 672
- US-A- 6 020 367
- DATABASE WPI Week 200349 Thomson Scientific, London, GB; AN 2003-517056 XP002554237 & JP 2003 089601 A (MENICON CO LTD) 28 March 2003 (2003-03-28)
- DATABASE WPI Week 199802 Thomson Scientific, London, GB; AN 1998-014641 XP002554238 & JP 09 278610 A (OFUTEKUSU KK) 28 October 1997 (1997-10-28)

## Description

### FIELD OF THE INVENTION

This invention relates to tissue adhesion prevention, especially the prevention of dehydration and oxidation upon exposure to air associated with surgery, and the prevention of complications such as tissue adhesion of postoperative organs.

### BACKGROUND ART

It is well-known that when tissues, including organs, are exposed to air due to incision in surgery that these tissues can become dehydrated and oxidized leading to their damage. The dehydration and oxidation of tissue, including organs, during surgery lead to their damage, which causes problems including postoperative tissue adhesion and the delay wound-healing.

Since especially adhesion often inhibits the normal movement of tissues, including organs, it is considered one of the serious post-surgery complications. For instance, the adhesion occurred during tendon surgery may cause dyskinesia. In addition, organ adhesion after intra-abdominal surgery may cause complications such as ileus, pain and sterility, and sometimes can cause problems so that the next surgery becomes very difficult.

Examples of current treatments for the prevention of tissue adhesion after surgery and the delay of wound-healing include a treatment in which saline solution is regularly perfused to exposed organs during surgery and treatment in which tissues such as organs are covered with gauze soaked with saline solution.

However, problems have arisen such that in case of perfusion of saline solution, it is difficult to maintain sufficient coverage of the organs with the fluid layer of saline solution, preventing the organs from exposure to the milieu that can cause oxidative stress. In case of covering organs with gauze soaked with saline solution, the gauze may interfere with the surgery operations. It was difficult to maintain conditions in which the whole surface of exposed tissue, including organs, is blocked from air during surgery.

Therefore, to solve these problems, adhesion preventives composed of chitin, adhesion preventives composed with bridged hyaluronic acid and drugs for prevention of delay of cure have been proposed (for instance, refer to Japanese patent publications No. 2948254 and 3420851).

However, the previous drugs described above lacked operationality, such as user-friendliness, because their component having an adhesion preventive function is a macromolecule and is mainly in film form, which is placed on the intended section or a viscous fluid that is applied to the intended section. Among substances for adhesion prevention or prevention of cure delay, water-soluble substances that and can easily cover the intended section have not been known.

Trehalose (C₁₂H₂₂O₁₁), a saccharide that has recently been used in various fields such as foods or cosmetics, is known to have very high hydrating properties compared to other saccharides. In addition, since trehalose has a similar structure to the cluster structure of H₂O, when administered to cell surfaces, it is considered to persist in glass (amorphous) condition after dehydration, stabilizing cells or proteins instead of H₂O and inhibiting these degenerations.

Novel indications for trehalose have been researched in various ways. For instance, drugs containing trehalose administered to a mucous membrane (such as Japanese patent publication no. 6-256219), nutrient solutions containing trehalose as a saccharide calorie (such as Japanese patent publications no. 6-72883 and no. 6-319486), solutions for transplanted organs containing trehalose (such as Japanese patent publication no. 6-40801), intraocular perfusion/washing agents, eye drops, eye ointments that contain trehalose and have cornea protection properties (such as Japanese patent publication no. 9-235233), association-forming agents between trehalose and unsaturated compounds used for the inhibition of chemical changes of unsaturated compounds (such as Japanese patent publication no. 2002-24832), inhibitors of lipolysis degradation containing trehalose (such as WO 2004/076602) have been proposed.

Solutions comprising trehalose in combination with other ingredients are known in the art. US-A-6,020,367 discloses solutions comprising trehalose in combination with anti-inflammatory agents, antioxidants, chelating agents and thickeners.

WO 93/15234 discloses solutions comprising trehalose and chelating agents.

WO 00/64254 discloses solutions comprising trehalose, antioxidants, plasma expanders and polysaccharides having lubricating properties.

WO 2004/098285 related to solutions comprising trehalose and polysaccharides which may act as viscosity adjusting agents or as polysaccharides having lubricating properties.

WO 2005/030248 relates to solutions comprising trehalose, antioxidants, chelating agents, dextrins, etc.

WO 00/72872 discloses solutions comprising trehalose, anti-inflammatory acting KGF-2 polypeptides and chelating agents.

EP-A-1 512 404 discloses solutions comprising trehalose and dextrans anti-inflammatory steroids and pectin.

WO 00/07634 discloses solutions comprising trehalose and microbicidal (antiseptic) compounds in a thickened and osmolarity adjusted solution.

DATABASE WPI Week 200349 Thomson Scientific, London, GB; AN 2003-517056 XP002554237 & JP 2003 089601 A (MENICON CO LTD) 28 March 2003 discloses solutions comprising trehalose and disinfecting compound in an isotonic solution.

DATABASE WPI Week 199802 Thomson Scientific, London, GB: AN 1998-014641 XP002554238 & JP 09 278610 A (OFUTEKUSU KK) 28 October 1997 discloses solutions compring trehalose in an antiseptic solution comprising chelating agents.

### DESCRIPTION OF THE INVENTION

The object of the invention is to provide a component for use in tissue adhesion prevention being applicable to general surgery in which covering condition during surgery is stable and convenient.

Said object is achieved by trehalose for use in tissue adhesion prevention, wherein the trehalose is in solution and is applied to local tissue. It was found that when a water solution of trehalose is sprayed on the surface of animal organs during surgery, postoperative adhesion is prevented and that the survival period of cells is prolonged even when the surface is dehydrated after having been sprayed with a water solution of trehalose.

Furthermore, it was surprisingly found that after spraying with a solution composed of trehalose and glycosyl-L-ascorbic acid and then dehydrating, surprisingly, the effects of both compounds were synergistically exerted and a stronger protection effect compared with the use of trehalose alone was shown.

Therefore, the invention is based on the discovery of a novel effect of trehalose, and solves the problems described above by trehalose for use in tissue adhesion prevention, wherein the trehalose is in a solution. The protection of the tissue is further improved when the solution for tissue adhesion prevention includes glycosyl-L-ascorbic acid.

The application of the solution for tissue adhesion prevention prevents dehydration of tissues, including wounds and organs, exposed to air during surgery, as well as inhibits oxidative stress leading to prevention of lipid peroxidation and lipid degradation, and, even when wounds and tissues are exposed to air during surgery, trehalose forms chelate with metals such as Fe and deactivates the metal ion that produces free radicals and, therefore, inhibits the production of free radicals, leading to the prevention of tissue adhesion after surgery and delay of wound cure.

It is preferred that trehalose for use in tissue adhesion prevention is in a solution which includes at least one or more antioxidants, chelates, antiseptics, hemostatics, anti-inflammatory agents, and polysaccharides, mucopolysaccharides, salts of polysaccharides and salts of mucopolysaccharides having lubricating properties.

When it includes antioxidants, the tissue protection property of trehalose and the free radical removal property of antioxidants are synergistically exerted to further prevent and inhibit the dehydration and oxidative stress of tissues, including wounds and organs, during surgery and to reduce the production of free radicals. When it includes chelates, it becomes possible to metabolize by conjugating divalent Fe ions with chelates, and to further reduce the production of free radicals.

As a result, it becomes possible to prevent more effectively the tissue adhesion after surgery and delay of wound cure.

When it includes at least one or more antiseptics, hemostatics and anti-inflammatory agents, it can be used as an antiseptic, hemostatic and anti-inflammatory agent during surgery or the treatment of wounds. It is, therefore, not necessary to administer drugs such as antiseptics, hemostatics and anti-inflammatory agents separately.

When it includes at least one or more polysaccharides, mucopolysaccharides, salts of polysaccharides, salts of mucopolysaccharides having lubricating properties, when applied for adhesion prevention to tissues, including wounds and organs, during surgery, it is possible to prevent not only dehydration due to air exposure, oxidative stress and the production of free radicals, but also mechanical damages such as microinjury during surgery.

It is well known that microinjury of organs often develops by touching the organs with hands during surgery. Because of the high biocompatibility and lubricating properties of trehalose in solution, it is possible to reduce damage due to touch with surgical gloves or surgical devices and to reduce mechanical damage of tissues including organs.

It is preferred that the antioxidant described above is a derivative of ascorbic acid.

By using such a composition, the tissue protection property of trehalose and the free radical removal property of the derivative of ascorbic acid are synergistically exerted to further prevent and inhibit the dehydration and oxidative stress of tissues, including wounds and organs, during surgery and to reduce the production of free radicals.

As a result, it becomes possible to prevent the tissue adhesion after surgery and delay of wound cure. In addition, it is suitable that trehalose is in a solution, wherein the viscosity of the solution for tissue adhesion prevention is adjusted by thickeners.

By using such a composition when the solution is administered to the surface of tissues, including wounds and organs, membranes covering wounds and tissues become thicker, ensuring that wounds and tissues are covered with sufficient thickness.

In addition, it is suitable that the osmolarity of the solution is adjusted by at least one or more isotonic electrolyte solutions, plasma expanders, extracellular replacement fluids, maintenance fluids or water.

By using such a composition, the solution can be isotonically adjusted and it is possible to transfer the nutrients of the composition to the tissues.

It is preferred when trehalose is in a solution which is provided as a form of perfusion fluid or spray fluid, a solution for spray or vaporization administration, a foam-like aerosol preparation, an injection solution for intravenous fluids, or an intravenous fluid.

When the solution comprising trehalose is provided as a perfusion fluid, it is possible to inhibit or prevent impairments of various tissues including adhesion by perfusing during or after surgery with the fluid of the invention.

In addition, when the solution is provided as a spray fluid, it is possible to cover the intended area by dispersing the solution widely and uniformly to the intended area during surgery. Impairment of tissues can be prevented due to a sufficient thickness of the solution. Compared with conventional methods in which viscous solutions with active ingredients of high-molecular weight are applied or film-form agents are placed on the intended sites, the solution comprising trehalose is able to prevent damage to tissues and the adhesion of tissues after surgery with a fair degree of certainty by ensuring that the intended areas are covered.

Furthermore, when trehalose is in a solution which is provided as a solution for spray or vaporization administration, the solution can be sprayed to the intended sites as a mist. It is possible to disperse the solution widely and uniformly as a refined mist during surgery to cover the intended site. Impairment of tissues can be prevented due to a sufficient thickness of the solution. When trehalose is in a solution which is provided as a foam-like aerosol preparation, it is possible to disperse it widely and uniformly to the intended sites during surgery to cover the intended sites. Impairment of tissues can be prevented due to a sufficient thickness of the solution.

When trehalose is in a solution which is provided as an injection solution for intravenous fluid(s), it is possible to administer the solution by means of including intravenous administration during at least one or more processes mentioned before, during and after surgery, to encourage rapid wound-healing.

By using trehalose in a solution for to be applied to local tissue, it is possible to prevent adhesion of local tissue or encourage rapid wound-healing.

The application of trehalose in a solution prevents dehydration of tissues, including wounds and organs, exposed to air during surgery and inhibits oxidative stress leading to the prevention of lipid peroxidation and lipid degradation. Even when wound and tissues are exposed to air during surgery, trehalose forms chelates with metals such as Fe and deactivates the metal ion that would cause the production of free radicals and therefore inhibits the production of free radicals, leading to the prevention of tissue adhesion after surgery and delay of wound cure.

In addition, since the solution comprising trehalose further contains derivatives of ascorbic acid, the tissue protection property of trehalose and the free radical removal property of the derivative of ascorbic acid are synergistically exerted to further prevent and inhibit the dehydration and oxidative stress of tissues including wounds and organs during surgery and the production of free radicals is reduced. As a result, it is possible to prevent the tissue adhesion after surgery and delay of wound cure.

### BRIEF DESCRIPTION OF THE DRAWINGS

### [Figure 1]

It is a graph showing changes in the cell survival rate due to air-drying in Example 1 of the invention.

### [Figure 2]

It is a graph showing the effects of each reagent on the cells in Example 1 of the invention.

Trehalose for use in tissue adhesion prevention is in a solution which is administered directly to tissues including wounds and organs during surgery. It can be used as a therapeutic agent that prevents delay of wound cure.

In this specification, hereinafter, "wound" refers to injury, and "tissue" to bodies' tissue such as skin, organs, muscles, nerves, cartilage, and bone.

Trehalose in solution can be applied to various animals, it is especially suitable to be used in mammalian species, preferably in humans.

Hereinafter, we describe one example of the invention in detail.

Trehalose in solution, according to this example, is administered to a tissue surface exposed during surgery. There is no special limitation due to the type of surgical intervention, it can be widely used in not only laparotomy or thoracotomy but also in neurosurgical procedures, orthopedic procedures relating to tendon or ligament, hepatic surgery, in gynecologic procedures etc..

The tissues exposed during surgery include, for instance, except wounds resulting from surgical incision and organs exposed by incision, muscles, nerves, cartilage and bone. It is also applicable for bone marrow exposed by orthopedic osteotomy. Furthermore, it has been reported that trehalose inhibits the development of nerve degenerative disorders in mice, therefore, it is applicable for cranial nerves and peripheral nerves exposed by neurosurgical procedures.

Trehalose is a non-reducing disaccharide formed by an α,α-1,1-glycoside bond between two α-glucose units.

Trehalose is a well-known substance, which was isolated from rye in 1832 for the first time. It can be found, for example, in animals, vegetables and microorganisms. It is present in a large amount in yeasts, such as bakers' yeast and brewers' yeast, and it is a saccharide often present in foods. Also, trehalose can be related to various organisms. Because trehalose is present in insects, such as Macrobiotus and Brachionus, and vegetables, such as Selaginella tamariscina, it is believed that it can survive under rigorous conditions, such as in a desert.

In this example, any of hydrated crystal trehalose, anhydrous crystal trehalose, carbohydrate solution containing trehalose, alpha, alpha-trehalose (in the narrow sense), alpha, beta-trehalose (neo-trehalose), beta, beta-trehalose (iso-trehalose) can be used, however, it is preferred to use hydrated crystal trehalose or anhydrous crystal trehalose composed of especially alpha, alpha-trehalose (alpha-D-glucopyranosyl-alpha-D-glucopyranoside).

In addition, only if it is the trehalose described above, any one produced from natural trehalose, starch, maltooligosaccharides, glucoses can be used.

As a solvent for the dissolution of trehalose, isotonic electrolyte fluids, plasma expanders or maintenance fluids or water can be used.

The isotonic electrolyte fluids refer to electrolyte preparations that are isotonic. The isotonic fluids refer to intravenous fluids that are isotonic, which means that the osmolarity is similar with the body fluid. As an isotonic electrolyte fluid, for instance, extracellular replacement fluid can be used.

The extracellular replacement fluid refers to intravenous isotonic electrolyte fluid used for the replacement or adjustment of extracellular fluid in case of depletion of extracellular fluid due to problems such as hemorrhage, diarrhea, and dehydration. Extracellular replacement fluids, for instance, can include physiological saline solution (0.9% saline solution), Ringer's acetate and Ringer's lactate.

Plasma expanders also refer to plasma extenders being used as intravenous fluids when proteins such as albumin in plasma are lost due to problems such as hemorrhage. They contain a high-molecular weight component such as dextran, starch and recombinant albumin. Dextran 40 preparation, dextran 70 preparation, hydroxyethyl starch (HES) preparation and modified gelatin preparation can be used as plasma expanders. Maintenance fluid is an intravenous fluid used for the replacement and maintenance of water/electrolyte when oral intake is impossible or insufficient. Starting fluid, maintenance fluid and high-concentration glucose added maintenance fluid can be used as maintenance fluid. Water acts as a solvent for the dissolution of trehalose.

Pure water or distilled water can be used. It is preferred to use sterile distilled water.

The concentration of trehalose in the solution is preferred when it is more than 10mM, preferably within 50 to 350mM.

When it is less than 10mM, the cell protection effect can not be expected. When it is more than 350mM, the solution becomes hypertonic.

To trehalose in solution at least one or more of the group, including antiseptics, hemostatics and anti-inflammatory agents may be added.

As antiseptics, for instance, well-known antiseptics such as povidone-iodine, potassium peroxomonosulfate, dimethyldidecyl ammonium chloride, as hemostatics, for instance, well known hemostatics such as thrombin, sodium alginate, as anti-inflammatory agents, for instance, non-steroid anti-inflammatory drugs are used.

Furthermore, to trehalose in a solution, antioxidants may be added.

For an antioxidant, water-soluble stable vitamin C can be used for example. The water-soluble stable vitamin C includes derivatives of ascorbic acid classified into pro-vitamin C agents such as glycosyl-L-ascorbic acid (hereinafter referred to as AA-2G) and ascorbic acid-2-phosphoric acid (AA-2P).

AA-2G also refers to ascorbic acid-2-glucoside that is present in nature and is degraded in vivo by alpha-glucosidase into L-ascorbic acid and D-glucose, developing similar physiological activity to L-ascorbic acid. AA-2G, even in high concentration, does not have cell toxicity and its promoting effect of collagen synthesis and promoting effect of cell proliferation and differentiation are significant, which lead to its frequent use not only in food or cosmetic fields but also in the culture of connective tissues such as fibroblasts.

As AA-2G, a series of 2-glucopyranosyl-L-ascorbic acid including 2-O-alpha-D-monoglucopyranosyl-L-ascorbic acid composed of a glycosidic linkage of L-ascorbic acid at the C-2 position and one or more D-glucose is preferably used. Also, AA-2G is not limited to glycosyl-L-ascorbic acid as organic acid, and can be any physiologically accepted mineral salts and salts of organic acid such as sodium salt, potassium salt, calcium salt, magnesium salt and ammonium salt that liberate glycosyl-L-ascorbic acid in aqueous media.

The concentration of AA-2G in the solution for tissue adhesion prevention should preferably be more than 0.01 mM, more preferably within 0.1 to 200mM. When it is less than 0.01mM the cell protection effect can not be expected.
As an antioxidant, minerals such as vitamin C, vitamin E, selenium, in vivo antioxidant enzymes such as SOD (super oxide dismutase), catalase, glutathione peroxidase, vegetable-derived antioxidants (SOD-like substances) that contain carotenoids that are lipid-soluble pigments of vegetables such as alpha-carotine, beta-carotine, gamma-carotine, lycopene, xanthophyll, and polyphenols that are contained in flower, leaf, bark, stem such as flavonoid, catechin, tannin, anthocyanin, isoflavon, quercetin, phycocyanobilin, and phycoerythrobilin can be used.

To trehalose in solution, furthermore, chelates may be added.

For chelates, DMPS (dimercaptopropanolsulfonate), DMSA (dimercaptosuccinate), ALA (alpha-lipoic acid), EDTA (ethylenediaminetetraacetic acid), L-glutamicdiacetic acid/tetrasodium (GLDA/4Na), sodium gluconate can be used.

Since chelates can be metabolized by conjugating divalent Fe ions, it is possible to prevent the production of free radical caused by Fe ions, leading to further more effective prevention of adhesion of tissues including organs after surgery and delay of wound cure.

To trehalose in solution, furthermore, at least one of polysaccharides, mucopolysaccharides and the salts thereof having lubricating properties may be added.

For polysaccharides and the salts thereof, for instance, polysaccharides containing carboxyl group or the water-soluble salts thereof or polysaccharides containing carboxyl group bridged by ion bonding or the water-soluble salts thereof can be used. For polysaccharides containing carboxyl group, carboxymethylcellulose, carboxymethylchitin, carboxymethylchitosan, carboxymethylstarch, alginic acid, pectin, carboxymethyldextran etc. can be used.

Mucopolysaccharides include, except for hyaluronic acid (HA), heparin, heparin sulfate, and chondroitin sulfate.

For water-soluble salts, sodium salts, alkali metal salts or alkali earth metal salts can be used.

In addition, it is preferred to use hyaluronic acid among polysaccharides, mucopolysaccharides and the salts thereof. Especially, mucopolysaccharides such as hyaluronic acid are known for their ability to gelate water hundreds to thousands of times its amount and to assure water retentivity, viscosity and lubricity to body fluid. If it is added to the solution for tissue adhesion prevention, when covering the surface of tissues such as wounds and organs with the solution because it assures lubricity to the cover layer having a preventive effect concerning mechanical damages to wounds and tissues.

The solution is adjusted to an osmolarity of from 200 to 450 Osm and a pH from 7 to 8 after mixing the substances described above.

The reason why the osmolarity is adjusted from 200 to 450 Osm is to adapt the osmolarity of the human body. In addition, the reason why the pH is from 7 to 8 is to prevent acidolysis of tissue cells of wounds or organs administered with the solution for tissue adhesion prevention.

For adjustment of osmolarity, colloid osmotic pressure adjustment agents such as hydroxyethylstarch and dextranstarch are used. The osmolarity is adjusted to low values considering condensation due to dehydration during surgery.

The solution is adjusted to a temperature during use which is near body temperature, that is, 35 to 38 degrees C. By this procedure, when the solution for tissue adhesion prevention is administered during surgery, the intended sites of tissues such as wounds and organs would not be cooled and it is possible to prevent cooling during surgery that causes postoperative pain.

The solution can be administered anytime before of the healing of wounds progresses, although it is preferable to administer it during surgery or immediately before the suturing of wounds. Also it can be administered continuously during surgery.

In addition, the solution is administered directly to tissue surfaces, including wounds and organs, exposed to air by incision during surgery. As a method of administration, after the solution for tissue adhesion prevention is impregnated with carriers such as gauze or nonwoven fabric, they may cover the tissue surface including wounds and organs. However, it is preferred to pour the solution directly to the tissue surface, including wounds and organs, or after composing the solution for tissue adhesion prevention as a spraying solution, it is sprayed with spraying devices.

By composing the solution for tissue adhesion prevention as spraying solution to be able to spray it to the intended sites, the solution can be sprayed widely and uniformly to the intended sites and the intended sites where impairment would be prevented can be covered with a sufficient thickness of the solution.

As spraying devices, both sprays with two fluid nozzles in which solution drops are carried with air or carbon dioxide and sprays with one fluid nozzle in which the solution is dispersed into tiny particles by pressure can be used. The one fluid nozzle does not use gas for providing drops. Therefore, use of the one fluid nozzle prevents dehydration or oxidation of the tissues, including wounds and organs, or the lowering of the temperature by the heat of evaporation. Therefore, it is the preferred spraying device for the prevention of damages of wounds and tissues.

The one fluid nozzle includes a hollow cone nozzle and a full cone nozzle in which the solution is sprayed out in cone shape by rotational flow, a nozzle of which the angle of the spraying cone can be changed, a flat nozzle in which the drops are sprayed out in plane shape, a solid nozzle in which the drops go straight forward, a particle spray in which the drops become tiny particles that are all applicable.

On the other hand, when the amount of polysaccharides, mucopolysaccharides and the salts thereof is increased to improve the preventative effect of mechanical damages of tissues, including wounds and organs, during surgery, it is recommended to use a spray with two fluid nozzles because the viscosity of the solution for tissue adhesion prevention is higher. When the solution for tissue adhesion prevention is poured directly to the tissue surface including wounds and organs, a certain viscosity is needed, therefore, the viscosity should be adjusted using thickeners such as gums, including: mucopolysaccharides or heteropolysaccharides, synthesized organic high-molecular weight compositions including polyvinylalchol or polyacrylic acid, cellulose derivatives and starch derivatives.

Also, it is preferred that the solution being applied to the local tissue is composed as a solution for spray or vaporization administration, and this solution for spray or vaporization administration is administered by spraying the mist atomized with devices such as a nebulizer and a vaporizer. By composing the solution as a solution for spray or vaporization administration to spray it to the intended sites in atomized mist state, the solution that works as the solution for tissue adhesion prevention can be sprayed widely and uniformly to the intended sites and the intended site where impairment would be prevented can be covered with a sufficient thickness of the solution.

For a nebulizer and vaporizer, any ultrasonic ones in which the solution is atomized by shaking with an ultrasonic oscillator, dropping ones in which drops are carried with gas or jet injection ones in which the solution is dispersed into tiny particles under pressure can be used.

For instance, a device provided heater, an ultrasonic oscillator, an air blasting fan, a spray orifice in which the solution for spray or vaporization administration is heated with a heater to a given temperature and is atomized by shaking with an ultrasonic oscillator, and the mist obtained by atomizing is sprayed from a spray orifice with an air blasting fan can be used. Also it is possible to use a two-phase vaporizer with the solution for tissue adhesion prevention composed of 2 solutions, that is, water and a concentrated solution of the solution for tissue adhesion prevention, equipped with a water tank with a heater and a concentrated solution tank with an ultrasonic oscillator. When the two-phase vaporizer is used, steam produced by heating with the heater and mist of the concentrated solution atomized with the ultrasonic oscillator are mixed in the pathway, and the mist of the solution for tissue adhesion prevention produced is sprayed out from the spray orifice.

Also, by composing the solution for tissue adhesion prevention as a foam-like aerosol preparation, and this foam-like preparation can be administered using a heater, a pressure device, an aerosol device provided nozzle. The foam-like aerosol preparation comprises known surfactants as already described.

Furthermore, in endoscopic surgery, which has been widely used in recent years, the problems of complications due to dehydration and oxidation of tissues including organs during surgery also have occurred. By applying trehalose for use in tissue adhesion prevention, wherein the trehalose is in a solution, it is possible to prevent complications after endoscopic surgery. By using a spray device with one fluid nozzle fixed to the top of an extension tube, the spray solution composed of the solution for tissue adhesion prevention can be sprayed to the tissues, including organs, involved in the surgery during or after endoscopic surgery. In addition, it is preferred if the extension tube of one fluid nozzle and the cable of the endoscopic device are fixed to one other, the operation of the spray device is facilitated because the materials inserted into the body during endoscopic procedures are unified.

Furthermore, the solution for tissue adhesion prevention can be composed as a perfusion fluid, an intravenous fluid, or an injection solution for intravenous fluids.

The perfusion fluid is an aqueous solution composed of dissolved trehalose and AA-2G in well known intravenous fluids such as saline solution and Ringer's solution, and is provided packed into intravenous fluid bags. The perfusion fluid is used by substituting and perfusing operation fields during or after surgery.

Intravenous fluid preparations are prepared by dissolving trehalose with well known tonicity agents such as sodium chloride, glycerin and well-known buffer fluids such as phosphate and acetate in well known solvents such as saline solution and Ringer's solution, and provided packed into intravenous fluid bags. The intravenous fluid preparations are used in at least one or more processes before, during and after surgery using means such as an intravenous drip.

Injection solutions for intravenous fluids are prepared by dissolving trehalose and AA-2G with well-known tonicity agents such as sodium chloride, glycerin and well known buffer fluid such as phosphate and acetate in well known solvents such as saline solution and Ringer's solution.

This injection solution is used as a mixed solution with well-known intravenous fluids such as glucose injection, xylitol injection, saline solution, dextran 40 injection, amino acid injection and Ringer's solution.

The injection solutions are used in at least one or more processes before, during and after surgery using means such as an intravenous drip to inject the intravenous fluid.

It has been found that during surgery, when tissues such as organs and wounds are exposed to oxygen in the air, dehydration, oxidative stress and free radicals occur leading to oxidative impairments such as membrane phospholipids impairment, protein impairment and DNA damages.

By administering the solution for tissue adhesion prevention directly on tissues, including organs and wounds, during surgery, trehalose prevents the dehydration of tissues and wounds and also prevents oxidative impairments due to oxidative stress during surgery. Also, trehalose forms chelates with metals such as Fe, deactivates metal ions that cause free radicals, and inhibits the production of free radicals. By preventing dehydration, oxidative impairments due to oxidative stress and production of free radicals, it is possible to prevent adhesion and delay of cure caused by oxidative impairments after surgery. In addition, since the solution for tissue adhesion prevention contains AA-2G, which is an antioxidant by adding radicals, it provides a synergistic effect in a way that wounds and exposed tissues can be protected more efficiently by the combination of trehalose and AA-2G, which have different mechanisms of action.

### [Example]

Hereinafter, we describe a specific example of the invention in detail.

### (Example 1)

As cells, HepG2 cells (human hepatoma-derived cell lines) were used.

For all reagents other than HepG2 cells, AA-2G, trehalose, DMEM media, FCS, neutral red, reagent grade chemicals were used. In addition, as sample solution in which HepG2 cells were activated, 4 kinds of solution; PBS (+) solution, 2.5mM AA-2G solution, 132mM trehalose solution, 2.5mM AA-2G + 132mM trehalose mixed solutions were prepared.

A reagent ascorbic acid-2-glucoside (AA2G; Hayashihara Biochemical Labs., Inc.) was dissolved in distilled water to obtain a 2.5mM AA-2G solution; and a highly purified hydrated crystal trehalose in powder (Reagent trehalose; Hayashihara Biochemical Labs., Inc.) was dissolved in distilled water to obtain a 132mM trehalose solution. The reagents ascorbic acid-2-glucoside and the highly purified hydrated crystal trehalose in powder were dissolved in distilled water to obtain a 2.5mM AA-2G + 132mM trehalose mixed solution. Then, pH and osmolarity of these 4 kinds of sample solutions were adjusted. The adjustment of osmolarity of the 2.5mM AA-2G solution, 132mM trehalose solution, 2.5mM AA-2G + 132mM trehalose mixed solution was performed by adjusting with sodium chloride of PBS (+) to be isotonic.

The pH and osmolarity of each sample after adjustment were as follows; pH 7.2 and 279mOsm for PBS (+) solution, pH 6.8 and 265mOsm for 2.5mM AA-2G solution, pH 7.2 and 283mOsm for 132mM trehalose solution and pH 7.0 and 282mOsm for 2.5mM AA-2G + 132mM trehalose mixed solution.

Then, HepG2 cells were seeded with 4 x 10⁴ cells/well each to four 12-well plates, and incubated in DMEM media (Nissui Pharmaceutical Co., Ltd) containing 10% FCS (Gibco) for 4 days. Then the DMEM media containing 10% FCS were replaced with the 4 kinds of sample solutions; PBS (+) solution, 2.5mM AA-2G solution, 132mM trehalose solution, 2.5mM AA-2G + 132mM trehalose mixed solution, and activated at 37 degrees C for 1 hour.

Two control plates and two test plates were prepared for cells activated with the 4 kinds of sample solutions, respectively. For each two test plates, one plate was air-dried for 5 minutes and another plate was air-dried for 3 minutes. The two control plates were not air-dried.

Then, cells of each plate were replaced with no serum DMEM media and incubated at 37 degrees C for 1 hour. After replacing with 0.02% neutral red (Wako Pure Chemical Industries, Ltd, hereinafter referred to NR), they were activated at 37 degrees C for 40 minutes. After removal of the NR solution and addition of PBS (+), they were left to stand at 37 degrees C for 40 minutes, PBS (+) was removed and they were air-dried for 5 minutes. NR extracts were added and they were left to stand at 37 degrees C for 10 minutes, then each sample was distributed over a 96-well plate and the absorbance at the wavelength of 570nm was measured.

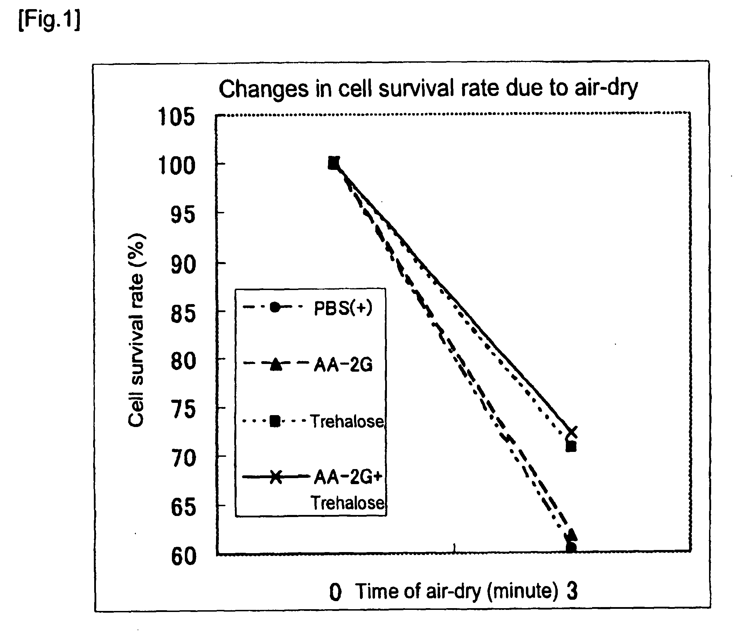

The absorbance of the control plates to which air-drying was not performed was considered to have a cell survival rate of 100%, and the cell survival rate of the test plates to which air-drying was performed for 3 minutes was calculated. The results are shown in the graph indicating changes in cell survival rate by air-drying in Figure 1.

The cell survival rates in the test plates to which air-drying was performed for 3 minutes were 60.4%, 61.8%, 70.7% and 72.3% for the PBS (+) solution, the 2.5mM AA-2G solution, the 132mM trehalose solution and the 2.5mM AA-2G + 132mM trehalose mixed solution, respectively.

The cell survival rate of the test plate to which air-drying was performed for 5 minutes for the PBS (+) solution was 54.9%.

As mentioned above, the cell protection effect was calculated for each sample solution considering the cell survival rate of the control plate as 100%, the cell protection effect was not observed for the 2.5mM AA-2G solution, and the cell protection effect was observed for the 132mM trehalose solution and the 2.5mM AA-2G + 132mM trehalose mixed solution when the plates were air-dried for 3 minutes.

Therefore, the cell protection effect from air-drying of these reagents is considered to be effective for slight dehydration of approximately 3 minutes.

The cell survival rates of the control plates for each sample solution to which air-drying was not performed were calculated.

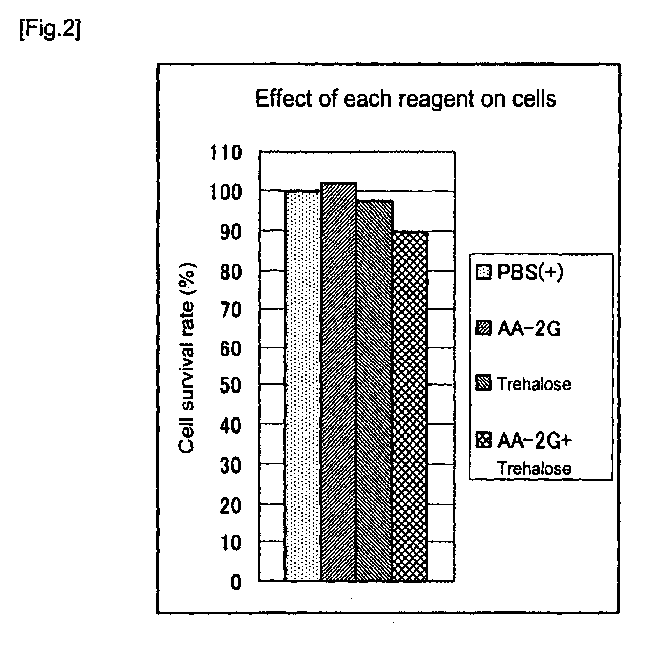

The results are shown in the graph indicating the effect of each reagent on cells in Figure 2. The cell survival rates in control plates, when the cell survival rate of the control plate for PBS (+) was considered to be 100%, were 101.6%, 97.6 % and 89.6% for 2.5mM AA-2G solution, 132mM trehalose solution and 2.5mM AA-2G + 132mM trehalose mixed solution, respectively.

Also, when the cell survival rate of the control plate for PBS (+) was considered to be 100%, the effects on cells from the 2.5mM AA-2G solution, the 132mM trehalose solution and the 2.5mM AA-2G + 132mM trehalose mixed solution were calculated. Each cell survival rate was 101. 5%, 97.7% and 89.7%, respectively, and it was found that adjustment of pH and osmolarity alleviated the effects on cells.

Since the protection mechanism of cells from dehydration and oxidation is known to be similar to the protection mechanism of the body's tissue from dehydration and oxidation, the water solution containing trehalose and antioxidant as active ingredients was confirmed to act as a solution for tissue adhesion prevention by this Example 1.

### (Example 2)

In this example, an abdominal adhesion model was prepared using rabbits by desiccating the surface of the intestines during abdominal surgery, and the preventative effect of intestine adhesion by trehalose was discussed.

As trehalose, powder trehalose (Reagent trehalose; Hayashihara Biochemical Labs., Inc.) and + NaCl were dissolved in sterile distilled water of 250mL as a 2% solution, sterilized by filtration with a filter (Millex; Millpore Co., Bedford) and poured into a sterile container and stored at room temperature. As test animals, five female New Zealand White rabbits weighing 2.85 to 3.2kg, aged 14 weeks (Std:NZW, Japan SLC, Inc., Shizuoka) were used. The rabbits were kept in an animal laboratory (room temperature of 24+/-1 degrees C, humidity of 30 to 70%) in the University of Tokyo, Graduate School of Agricultural and Life Sciences Veterinary Medical Center. The rabbits were acclimatized for 1 week after purchase, and during this period blood test (complete blood count, BUN, Cre, ALT, ALP) and general x-ray test were performed to confirm that abnormality was not present. The experiment of this example was performed according to the Manual of Animal Experiment of the University of Tokyo.

The experiment of this example was performed as follows.
As anesthesia, 5mg/kg of ketamine (Ketalal 50; Sankyo Lifetech Co., Ltd., Tokyo) and 0.1mg/kg of medetomidine (Domitol; Meiji Seiyaku Co., Ltd., Tokyo) were intramuscularly injected as preoperative medication, and after intubation maintained with isoflurane (Isoflu; Dainippon Pharmaceutical Co., Ltd., Osaka). Before the operations, 10mg/kg of enrofloxacin (Baytril; Bayer Medical Ltd., Tokyo) was subcutaneously administered as antibiotics, and during the operations 10mL/kg/hr of lactated Ringer's solution (Fuso; Fuso Pharmaceutical Industries, Ltd., Osaka) was intravenously administered. After the operations, a single dose of 20 g/kg of buprenorphine (Lepetan; Otsuka Pharmaceutical Co., Ltd., Tokyo) as analgesics was subcutaneously administered. During anesthesia, ECG, ETCO2 and SpO2 were monitored and maintained within their normal variations.

Three rabbits among the five rabbits were randomized to the control group, and the other two rabbits were classified into the trehalose group. For the five rabbits, after disinfection of the operative field, abdominal median incision was performed and intestines were exposed as far as possible and serum was wiped with gauze. For the control group (three rabbits) they were left desiccated and for the trehalose group (two rabbits) whole intestines were sprayed with trehalose several times. During this period, ovariohysterectomy was performed according to a given method. The time period of laparotomy was 30 to 60 minutes for both groups. The 3-0 monofilament nylon fiber (Bear surgical suture; Bear Medic Corporation, Chiba) was used for ovariohysterectomy and closure of the abdominal wall, and the 4-0 monofilament polyglyconate fiber (MAXON; Syneture Co., Ltd., Connecticut) was used for subcutaneous tissues and a stapler (Manipler AZ-35W; Mani Inc., Tochigi) was used for skin suture.

After the operations, general condition and wounds were observed and they were killed by overdose of pentobarbital after 2 weeks. The adhesion status in peritoneal cavity was macroscopically observed and recorded as well as tissues of adhesion sites were removed as pathohistological test material and fixed in formalin.

The clinical findings after 2 weeks and the macroscopic findings of adhesion status after laparotomy of each case are shown in Table 1.

### [Table 1]

### Clinical findings after 2 weeks and macroscopic findings after laparotomy

**[Table 1]**

| Clinical findings after 2 weeks and macroscopic findings after laparotomy | | | |
|---|---|---|---|
| Rabbit no. | Clinical findings | Findings after laparotomy | |
| Control group | | Adhesion sites in intestines | Adhesion of stump of uterus |
| 1. | No abnormal findings | 3 sites of adhesion | Present |
| 2. | No abnormal findings | 5 sites of adhesion | Present |
| 3. | Subcutaneous seroma | Not less than 5 sites of adhesion | Present |

| Trehalose group | | | |
|---|---|---|---|
| 1. | Partial dehiscence of skin suture | 2 sites of adhesion (Suspected) | Present |
| 2. | No abnormal findings | No significant adhesion sites | Present |

No clinical abnormality was observed in all cases at 2 weeks after operation. However, moderate subcutaneous retention of serum just under the surgical wounds was observed in 1 case in the control group. The result of bacterial cultures of this serum was negative. In addition, a part of the skin suture site was removed and showed ulcer in 1 case in the trehalose group.

For adhesion, a significant adhesion with surrounding tissue at suture of uterus stumps by ovariohysterectomy was observed in all cases in both groups. For adhesion of intestines, multiple adhesions (more than 3 sites) of mild to moderate degree were confirmed in all 3 rabbits of the control group. In the trehalose group, 2 sites of mild adhesion were suspected in 1 rabbit, however, they can be considered as normal bonds of the intestines. In the remaining 1 rabbit, no significant adhesion was macroscopically observed.

This Example shows that a spray of trehalose would be effective for the prevention of abdominal adhesion due to desiccation. Trehalose has high hydrating properties, and trehalose in amorphous state is considered to prevent adhesion by covering membrane of the intestines and stabilizing cells.

### (Example 3)

In this example, an abdominal adhesion model was prepared using rabbits by desiccating the surface of the intestines during abdominal surgery, and the preventive effect on intestine adhesion by trehalose was discussed.
As trehalose, powder trehalose (Reagent trehalose; Hayashihara Biochemical Labs., Inc.) was dissolved in sterile distilled water of 250mL as a 7% solution, sterilized by filtration with a filter (Millex; Millpore Co., Bed ford) and poured into a sterile container and stored at room temperature.
As test animals, 20 female New Zealand White rabbits weighing 2.6 to 3.2kg, aged 14 weeks (Std:NZW, Japan SLC, Inc., Shizuoka) were used. The rabbits were kept in the animal laboratory (room temperature of 24+/-1 degrees C, humidity of 30 to 70%) in the University of Tokyo, Graduate School of Agricultural and Life Sciences Veterinary Medical Center. The rabbits were acclimatized for 1 week after purchase, and during this period blood tests (complete blood count, BUN, Cre, ALT, ALP) and general x-ray tests were performed to confirm that abnormality was not present. The experiment of this example was performed according to the Manual of Animal Experiment of the University of Tokyo.

The experiment of this example was performed as follows.
As anesthesia, 5mg/kg of ketamine (Ketalal 50; Sankyo Lifetech Co., Ltd., Tokyo) and 0.1mg/kg of medetomidine (Domitol; Meiji Seiyaku Co., Ltd., Tokyo) were intramuscularly injected as preoperative medication, and after intubation maintained with isoflurane (Isoflu; Dainippon Pharmaceutical Co., Ltd., Osaka). Before operation, 10mg/kg of enrofloxacin (Baytril; Bayer Medical Ltd., Tokyo) was subcutaneously administered as antibiotics, and during operation 10mL/kg/hr of lactated Ringer's solution (Fuso; Fuso Pharmaceutical Industries, Ltd., Osaka) was intravenously administered. After operation, a single dose of 20 g/kg of buprenorphine (Lepetan; Otsuka Pharmaceutical Co., Ltd., Tokyo) as analgesics was subcutaneously administered. During anesthesia, ECG, ETCO2 and SpO2 were monitored and maintained within their normal variations.

For the 20 rabbits, after disinfection of the operative field, abdominal median incision was performed and intestines were exposed as far as possible and serum was wiped with gauze. Total ovariohysterectomy was performed according to a given method. Four sections of ovarion arteries and veins were severed after being ligated with 4-0 mono filament polyglyconate suture (MAXON; Syneture Co., Ltd., Connecticut). Also, uterovaginal stump were sutured with 5-0 mono filament polyglyconate suture (MAXON; Syneture Co., Ltd., Connecticut), the 3-0 mono filament polyglyconate suture (MAXON ; Syneture CO., Ltd., Connecticut) was used for closure of the abdominal wall, and the 4-0 mono filament polyglyconate suture (MAXON; Syneture Co., Ltd., Connecticut) was used for subcutaneous tissues and a stapler (ManiplerAZ-35W;Mani Inc., Tochigi) was used for skin suture.

Ten rabbits among the twenty rabbits were randomized to the control group, and the other ten rabbits were classified into the trehalose group.

The control group (10 rabbits) was left desiccated. The time period of laparotomy was 60 minutes. For the trehalose group, a trehalose solution was sprayed over the whole abdominal cavity every 15 minutes (0 minutes, 15 minutes 30 minutes, 45 minutes, 60 minutes after starting the operation). The time period of laparotomy was 60 minutes. After the operations, the general conditions and wounds were observed and the rabbits were euthanized by excessive administration of pentobarbital two weeks after the operations.

The adhesion status in peritoneal cavity was macroscopically observed and recorded as well as the tissues of the adhesion sites were removed as pathohistological test materials and fixed in formalin. The clinical findings after 2 weeks and the macroscopic findings of adhesion status after laparotomy of each case are shown in Table 2.

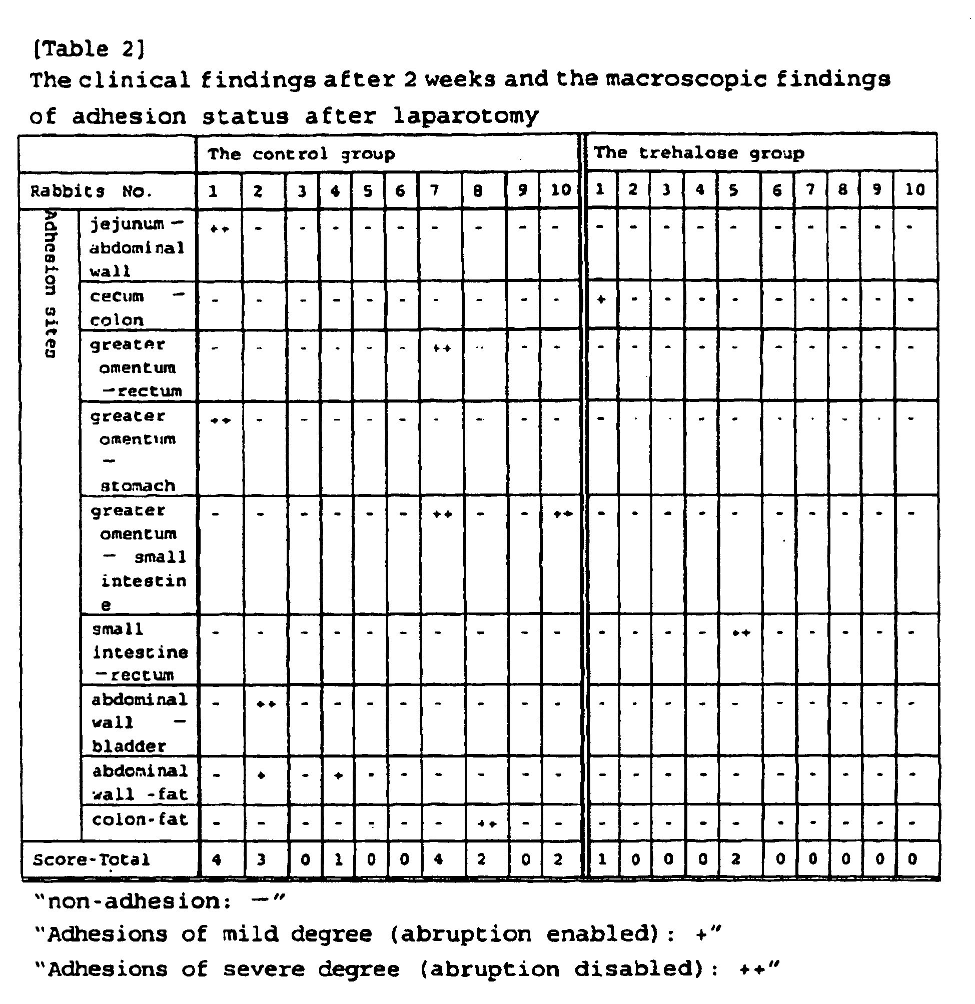

No clinical abnormality was observed in all cases at 2 weeks after the operations.

For adhesion, obvious adhesions with surrounding organ tissues at the suture of uterovaginal stumps, which are surgical sites of ovariohysterectomy, and vascular ligations were observed in all cases in the control group. For adhesion between abdominal organs of desiccated intestine surfaces, adhesions of mild and severe degree were confirmed in 6 of 10 control cases and 2 of 10 in trehalose cases. For adhesion scores between organs, the trehalose group achiered at +3,wheras +16 in the control group. The prevention ratio was 81%(Wilcoxon rank-sum test: at p<0.05).

This Example shows that trehalose can obviously prevent and reduce adhesions and would be effective for abdominal adhesion due to desiccation.

Trehalose has high hydrating properties, and is considered to be able to prevent adhesion by covering serous membranes of intestines and stabilizing cells.

Spraying of the solution made it possible to diffuse and penetrate serous membranes organ. Whole organs could be covered by trehalose.

## Claims

1. Trehalose for use in tissue adhesion prevention, wherein the trehalose is in solution and is applied to local tissue.

2. Trehalose for use in tissue adhesion prevention according to claim 1, **characterized in that** said solution includes at least one or more antioxidants optionally selected from derivatives of ascorbic acid, chelates, antiseptics, hemostatics, anti-inflammatory agents, and polysaccharides, mucopolysaccharides and salts of polysaccharides, salts of mucopolysaccharides having lubricating properties.

3. Trehalose for use in tissue adhesion prevention according to claim 1 or 2, **characterized in that** the viscosity of said solution is adjusted by thickeners.

4. Trehalose for use in tissue adhesion prevention according to any of claims 1 to 3, **characterized in that** the osmolarity of said solution is adjusted by at least one or more among isotonic electrolyte fluids, plasma expanders, extracellular replacement fluids, maintenance fluids and water.

5. Trehalose for use in tissue adhesion prevention according to any of claims 1 to 4, **characterized in that** said solution is provided as any form of perfusion fluid, spray fluid, solution for spray or vaporization administration, foam-like aerosol preparation, injection solution for intravenous fluids, or intravenous fluid.

## Patentansprüche

1. Trehalose zur Verwendung bei der Vorbeugung von Gewebeadhäsion, wobei die Trehalose in Lösung vorliegt und auf ein lokales Gewebe angewendet wird.

2. Trehalose zur Verwendung bei der Vorbeugung von Gewebeadhäsion gemäß Anspruch 1, **dadurch gekennzeichnet, dass** die Lösung mindestens ein oder mehrere Antioxidantien einschließt, gegebenenfalls ausgewählt aus Derivaten der Ascorbinsäure, Chelaten, Antiseptika, Hämostatika, entzündungshemmenden Mitteln, und Polysacchariden, Mucopolysacchariden und Salzen von Polysacchariden, Salzen von Mucopolysacchariden mit Gleiteigenschaften.

3. Trehalose zur Verwendung bei der Vorbeugung von Gewebeadhäsion gemäß Anspruch 1 oder 2, **dadurch gekennzeichnet, dass** die Viskosität der Lösung durch Verdickungsmittel eingestellt wird.

4. Trehalose zur Verwendung bei der Vorbeugung von Gewebeadhäsion gemäß einem der Ansprüche 1 bis 3, **dadurch gekennzeichnet, dass** die Osmolarität der Lösung durch mindestens eines oder mehrere aus isotonen Elektrolytflüssigkeiten, Plasmaexpandern, extrazellulären Ersatzflüssigkeiten, Aufrechterhaltungsflüssigkeiten und Wasser eingestellt wird.

5. Trehalose zur Verwendung bei der Vorbeugung von Gewebeadhäsion gemäß einem der Ansprüche 1 bis 4, **dadurch gekennzeichnet, dass** die Lösung in jeglicher Form von Perfusionsflüssigkeit, Sprühflüssigkeit, Lösung zur Verabreichung durch Spray oder Zerstäubung, schaumartiger Aerosolzubereitung, Injektionslösung für intravenöse Flüssigkeiten oder intravenöser Flüssigkeit bereitgestellt wird.

## Revendications

1. Tréhalose pour utilisation dans la prévention de l'adhésion tissulaire, le tréhalose étant en solution et est appliqué à un tissu local.

2. Tréhalose pour utilisation dans la prévention de l'adhésion tissulaire selon la revendication 1, **caractérisé en ce que** ladite solution contient au moins un ou plusieurs antioxydants éventuellement choisis parmi les dérivés d'acide ascorbique, les chélates, les antiseptiques, les hémostatiques, les agents anti-inflammatoires, et les polysaccharides, les mucopolysaccharides et les sels de polysaccharides, les sels de mucopolysaccharides ayant des propriétés lubrifiantes.

3. Tréhalose pour utilisation dans la prévention de l'adhésion tissulaire selon la revendication 1 ou 2, **caractérisé en ce que** la viscosité de ladite solution est ajustée par des épaississants.

4. Tréhalose pour utilisation dans la prévention de l'adhésion tissulaire selon l'une quelconque des revendications 1 à 3, **caractérisé en ce que** l'osmolarité de ladite solution est ajustée par au moins un ou plusieurs agents choisi parmi les fluides électrolytiques isotoniques, les succédanés du plasma, les fluides de remplacement extracellulaire, les fluides de maintien et l'eau.

5. Tréhalose pour utilisation dans la prévention de l'adhésion tissulaire selon l'une quelconque des revendications 1 à 4, **caractérisé en ce que** ladite
solution se présente sous une forme quelconque parmi un fluide pour perfusion, un fluide pour pulvérisation, une solution pour administration par pulvérisation ou vaporisation, une préparation pour aérosol de type mousse, une solution injectable pour fluides intraveineux, ou un fluide intraveineux.
